(19) Europäisches Patentamt
European Patent Office
Office européen des brevets

(11) Publication number: **0 091 305**
**B1**

(12) **EUROPEAN PATENT SPECIFICATION**

(45) Date of publication of patent specification: **21.10.87**

(21) Application number: **83301882.3**

(22) Date of filing: **31.03.83**

(51) Int. Cl.[4]: **C 12 P 7/02,** C 12 P 7/18, C 12 P 5/02, C 12 P 17/02, C 07 C 31/34, C 07 C 31/36, C 07 C 31/42 // A01N31/02, C09K21/08

(54) **Method for producing vicinal, heterogeneous dihalogenated products, their use, and novel products.**

(30) Priority: **02.04.82 US 364920**

(43) Date of publication of application:
**12.10.83 Bulletin 83/41**

(45) Publication of the grant of the patent:
**21.10.87 Bulletin 87/43**

(84) Designated Contracting States:
**AT BE CH DE FR GB IT LI LU NL SE**

(56) References cited:
**EP-A-0 007 176**
**FR-A-2 359 101**

**BIOCHEMICAL AND BIOPHYSICAL RESEARCH COMMUNICATIONS, vol. 110, no. 3, 10th February 1983, pages 880-883, New York, USA T.D. LEE et al.: "Neighboring group migration in enzyme-mediated halohydrin formation"**

(73) Proprietor: **CETUS CORPORATION**
**1400 Fifty-Third Street**
**Emeryville California 94608 (US)**

(72) Inventor: **Geigert, John**
**231 Southbrook Place**
**Clayton California 94517 (US)**
Inventor: **Neidleman, Saul Lewis**
**533 Hilltop Crescent**
**Oakland California 94618 (US)**

(74) Representative: **Bizley, Richard Edward et al**
**BOULT, WADE & TENNANT 27 Furnival Street**
**London EC4A 1PQ (GB)**

(56) References cited:
**BIOCHEMISTRY, vol. 7, no. 9, September 1968, pages 3213-3218, Easton, P.A., USA C.E. CASTRO et al.: "Biodehalogenation. Epoxidation of halohydrins, epoxide opening and transhalogenation by a Flavobacterium sp."**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 091 305 B1**

Courier Press, Leamington Spa, England.

**Description**

This invention relates generally to an enzymatic process for making useful commercial products from alkenes and alkynes, to the uses of such products and to certain novel products of such a process. More particularly, the invention relates to an improved process for the production of vicinally, heterogeneously dihalogenated products from alkenes and alkynes wherein an enzyme is used to effect the reaction. Also more particularly, the invention relates to an improved process for the production of fluorinated products from alkenes and alkynes wherein an enzyme is used to effect the reaction.

Vicinally, heterogeneously dihalogenated products are useful as solvents, chemical intermediates, refrigerants, fumigants, and in many other ways. Vicinal chloroiodo compounds (I) and vicinal bromochloro compounds (II) have found application in the agricultural chemical field as soil fumigants and pesticidal agents (U.S. Patent No. 3,576,893, Baird Jr. et al, 1971; Roth et al, *J. Economic Entomology 63*, 496 (1970)). In addition, the vicinal bromochloro group (II) imparts flame retarding capability into plastics and fibers (Pol. Patent No. 105,589, Dul et al, 1980). 1,2-Chlorofluorethane (III) is useful both as a refrigerant and as a solvent for recovering coal from mixtures (U.S. Patent No. 4,147,733, Fiske et al, 1979; U.K. Patent Application No. 2,028,173 A, Keller et al, filed Aug. 3, 1979).

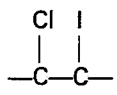

I

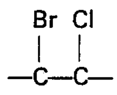

II

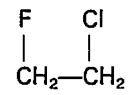

III

Owing to differences in chemical reactivity of the different carbon-halogen bonds, one halogen atom may be selectively removed either chemically or enzymatically from these vicinal, heterogeneous dihalogenated products. Thermal decomposition or photolysis of 1,2-bromochloroethane (IV) preferentially yields vinyl chloride (V) over vinyl bromide (VI) (Johnson et al, *J. Physical Chemistry 71*, 4366 (1967)):

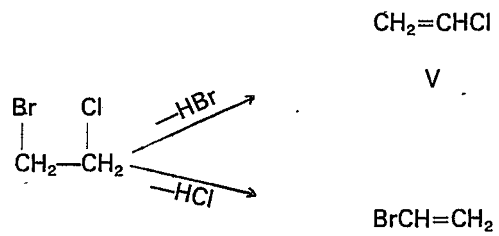


V

IV VI

Catalytic hydrogenation preferentially dehydrohalogenates in the following order: I>:Br>:Cl>F; thus, permitting the production of fluorine-containing products from the vicinal, heterogeneous dihalogenated products (Rylander, *Catalytic Hydrogenation in Organic Synthesis,* 235 (1979)):

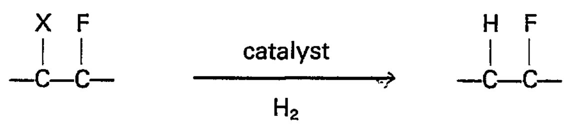


X=Cl, Br, I.

Fluorine imparts biological effects useful in drugs and other bioactive compounds.

Known processes for producing the vicinal dihalogenated products from alkenes and alkynes typically involve the addition of alkene or alkyne and free heterogeneous halogen in a reactor under controlled conditions. The use of free heterogeneous halogen in these processes requires expensive control procedures and equipment to prevent loss of this toxic and corrosive reactant. Also, the use of free heterogeneous halogen is now preferably avoided because of the energy-intensive process employed for its production.

An improved process is provided by the present invention for producing vicinally, heterogeneously dihalogenated products from alkenes and alkynes. The enzymatic halogenating process of the present invention has several advantages over the present state of the art for producing vicinally, heterogeneously dihalogenated products from alkenes and alkynes, including the following: The use of inexpensive, less dangerous, inorganic halides, rather than elemental halogen or expensive reagents, i.e., chloride ion plus bromide ion rather than bromine chloride, fluoride ion plus iodide ion rather than iodine fluoride or antimony trifluoride and iodine; the use of ambient temperature; and the use of standard or close to standard atmospheric pressure.

In addition to proceeding favourably at room temperature, this enzymatic process involves the use of dilute $H_2O_2$, not necessarily purified. The $H_2O_2$ may be added directly or generated *in situ* by an enzymatic or chemical reaction. This reduces the cost of the $H_2O_2$ as compared to the cost of concentrated, purified material; increases the safe usage of the substance; and extends the life of the halogenating enzyme.

Very generally, the low cost method of the invention produces vicinally, heterogeneously dihalogenated products from alkenes and alkynes by providing in a reaction vessel a mixture of haloperoxidase, an oxidizing agent and two different halide ions, both halide ions being capable of being used by the haloperoxidase and the total concentration of both halide ions being greater than 2000 mM. An alkene or alkyne susceptible to dihalogenation by the haloperoxidase is introduced into the vessel and maintained in contact with the reaction mixture for a sufficient period of time to convert the alkene or alkyne to the desired vicinally, heterogeneously dihalogenated product.

The present invention is based on the discovery that the group of enzymes classified as haloperoxidases acts upon alkenes and alkynes to produce vicinally, heterogeneously dihalogenated products. These products are characterized by the structural formula:

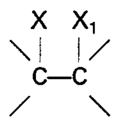

and

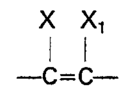

where X and $X_1$ are selected from a group consisting of either fluoride, chloride, bromide or iodide, but where $X \neq X_1$.

Prior art processes teach that haloperoxidase produces vicinal, *homogeneous* dihalogenated products from alkenes and alkynes (U.S. Patent Application Serial No. 330,157, filed December 14, 1981, Geigert et al US—A—4426449).

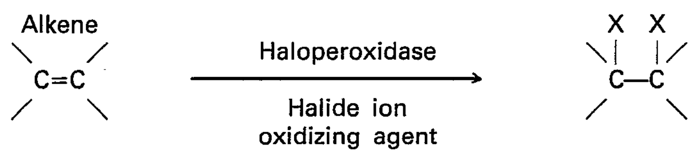


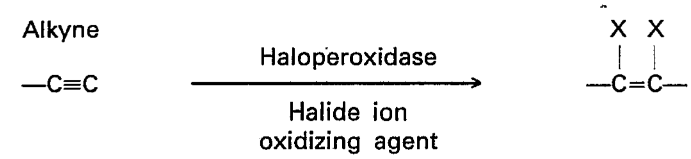


However, prior art processes do not teach that vicinal, *heterogeneous* dihalogenated products can be produced from alkenes and alkynes by haloperoxidase:

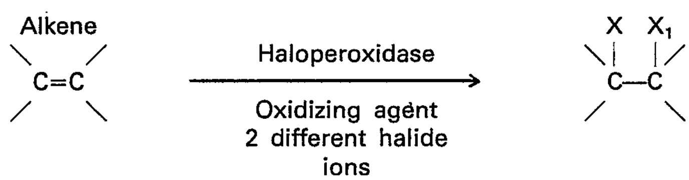


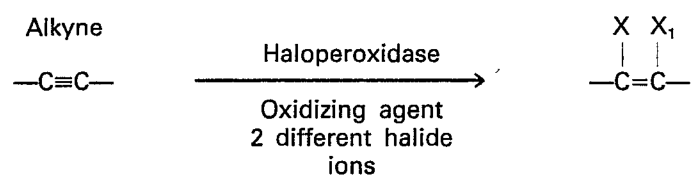


(where $X \neq X_1$).

Moreover, from these prior art processes it was not obvious to run the alkene and alkyne reactions in the presence of two different halide ions. Morrison et al (*Ann. Rev. Biochem. 45*, 861 (1976)) state that chloroperoxidase is the only one of the haloperoxidases studied that is known to be influenced by the presence of halide ion mixtures. Both chloride and bromide in concentrations up to 0.1 M greatly enhance iodination of thyroglobulin. Although this stimulation was observed, only homogeneous monohalogenated product was observed. Hager et al (*J. Biol. Chem. 241*, 1769 (1966)) state that fluoride ion when present with chloride ion inhibits the chlorination of monochlorodimedon by chloroperoxidase. Bakkenist et al (*Biochim. Biophys. Acta 613*, 337 (1980)) reported the same effect of fluoride ion on the

bromination of monochlorodimedon by myeloperoxidase. But in none of these reports was there any evidence presented for heterogeneous dihalogen addition. Therefore, it was not obvious that haloperoxidase would produce vicinal, heterogeneous dihalogenated products from alkenes and alkynes when the enzymatic reactions were run in the presence of two different halide ions.

In accordance with this invention, a process has been developed for the production of vicinally, heterogeneously dihalogenated products.

Prior art teaches that haloperoxidases can incorporate only certain halides (Morrison et al, *Ann. Rev. Biochem. 45*, 861 (1976)):

| Haloperoxidase | Halides that can be incorporated | Halides that cannot be incorporated |
|---|---|---|
| Myeloperoxidase | $Cl^-$, $Br^-$, $I^-$ | $F^-$ |
| Chloroperoxidase | $Cl^-$, $Br^-$, $I^-$ | $F^-$ |
| Lactoperoxidase | $Br^-$, $I^-$ | $F^-$, $Cl^-$ |
| Bromoperoxidase | $Br^-$, $I^-$ | $F^-$, $Cl^-$ |
| Thyroid peroxidase | $I^-$ | $F^-$, $Cl^-$, $Br^-$ |
| Horseradish peroxidase | $I^-$ | $F^-$, $Cl^-$, $Br^-$ |

In accordance with this invention, a process has been developed for the production of vicinally, heterogeneously dihalogenated products that contain halogen atoms not previously demonstrated to be incorporated by haloperoxidases.

The present invention includes the new compounds 2 - bromo - 3 - iodo - 1 - propanol; 3 - bromo - 2 - iodo - 1 - propanol; 3 - fluoro - 2 - iodo - 1 - propanol; 2 - fluoro - 3 - iodo - 1 - propanol and 2 - bromo - 3 - chloro - 1,4 - butanediol.

The alkenes useful in the process of the invention can be broadly defined as any hydrocarbon containing a carbon-to-carbon double bond, represented by the following structural formula:

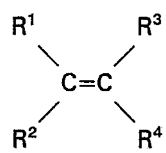

wherein each of $R^1$, $R^2$, $R^3$ and $R^4$ is selected from a group consisting of:

(1) hydrogen
(2) a straight chain
(3) a branched chain } saturated or unsaturated hydrocarbon radical
(4) a cyclic

Representative alkenes are:

| Alkene | $R^1$ | $R^2$ | $R^3$ | $R^4$ |
|---|---|---|---|---|
| Ethylene | H | H | H | H |
| Propylene | $CH_3$ | H | H | H |
| Butene-1 | $C_2H_5$ | H | H | H |
| Pentene-1 | $C_3H_7$ | H | H | H |
| Octene-1 | $C_6H_{13}$ | H | H | H |
| Decene-1 | $C_8H_{17}$ | H | H | H |
| Dodecene-1 | $C_{10}H_{21}$ | H | H | H |
| Isobutylene | $CH_3$ | $CH_3$ | H | H |
| Cis-butene-2 | $CH_3$ | H | $CH_3$ | H |
| Trans-butene-2 | $CH_3$ | H | H | $CH_3$ |
| 2-Methyl-butene-2 | $CH_3$ | $CH_3$ | $CH_3$ | H |
| 1,3-Butadiene | $CH_2{=}CH$ | H | H | H |
| 1,4-Pentadiene | $H_2C{=}CHCH_2$ | H | H | H |
| Isoprene | $H_2C{=}C(CH_3)$ | H | H | H |
| 1,7-Octadiene | $H_2C{=}CH(CH_2)_4$ | H | H | H |

The broad definition of alkenes includes alkenes where $R^1$, $R^2$, $R^3$, and/or $R^4$ can be an aromatic or heteroatom-containing group, provided that the substituents are inert to the prescribed reaction conditions, or do not deactivate the normally reactive carbon-to-carbon double bond.

Representative alkenes containing such aromatic or heteroatom groups are:

| Alkene | $R^1$ | $R^2$ | $R^3$ | $R^4$ |
|---|---|---|---|---|
| Allyl chloride | $ClCH_2$ | H | H | H |
| Allyl bromide | $BrCH_2$ | H | H | H |
| Allyl alcohol | $HOCH_2$ | H | H | H |
| 2-Butene-1,4-diol | $HOCH_2$ | H | $HOCH_2$ | H |
| 2-Buten-1-ol | $HOCH_2$ | H | H | $CH_3$ |
| 3-Buten-1-ol | $HO(CH_2)_2$ | H | H | H |
| 4-Penten-1-ol | $HO(CH_2)_3$ | H | H | H |

The R groups can also be connected to form a cyclic ring. A representative cyclic alkene is 2-cyclohexen-1-ol (VII).

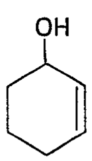

VII

The alkynes useful in the process of the invention can be broadly defined as any hydrocarbon containing a carbon-to-carbon triple bond, represented by the following structural formula:

$$R^1—C≡C—R^2$$

wherein each of $R^1$ and $R^2$ is selected from a group consisting of:

(1) hydrogen
(2) a straight chain
(3) a branched chain } saturated or unsaturated hydrocarbon radical
(4) a cyclic.

Representative alkynes are:

| Alkyne | $R^1$ | $R^2$ |
|---|---|---|
| Methyl acetylene | $CH_3$ | H |
| Ethyl acetylene | $CH_3CH_2$ | H |
| 1-Phenyl-1-propyne | $C_6H_5$ | $CH_3$ |
| Propargyl alcohol | $HOCH_2$ | H |
| 2-Butyne-1,4-diol | $HOCH_2$ | $HOCH_2$ |
| 3-Butyn-1-ol | $HO(CH_2)_2$ | H |

The present invention makes use of haloperoxidase enzymes. Such enzymes include chloroperoxidase derived from the microorganism *Caldariomyces fumago*, bromoperoxidase derived from algae, lactoperoxidase derived from milk, thyroid peroxidase derived from thyroid, myeloperoxidase derived from leukocytes, and horseradish peroxidase derived from horseradish. Certain of these haloperoxidases are commercially available.

The preferred haloperoxidase depends upon the products desired. The halides that the given haloperoxidases can use are listed below:

| Haloperoxidase | Halides, $X^-$ | Halides, $X_1^-$ |
|---|---|---|
| Myeloperoxidase | $Cl^-$, $Br^-$, $I^-$ | $F^-$, $Cl^-$, $Br^-$, |
| Chloroperoxidase | $Cl^-$, $Br^-$, $I^-$ | $F^-$, $Cl^-$, $Br^-$, |
| Lactoperoxidase | $Br^-$, $I^-$ | $F^-$, $Cl^-$, $Br^-$, |
| Bromoperoxidase | $Br^-$, $I^-$ | $F^-$, $Cl^-$, $Br^-$, |
| Thyroid peroxidase | $I^-$ | $F^-$, $Cl^-$, $Br^-$, |
| Horseradish peroxidase | $I^-$ | $F^-$, $Cl^-$, $Br^-$ |

(where $X^- \neq X_1^-$).

For ease of discussion, various aspects of the present invention are described below particularly, but not exclusively, in connection with the use of the preferred peroxidase, chloroperoxidase, derived from *Caldariomyces fumago* (ATCC 16373). The microorganism, *Caldariomyces fumago*, may be grown as a static or agitated, submerged culture in Czapek-Dox medium at room temperature for 3 to 10 days by conventional methods. The halogenating enzyme, chloroperoxidase, is prepared from an aqueous homogenate of the mycelial pads of the microorganism grown under static conditions or from the filtrate of the microorganism grown under static or agitated submerged culture conditions. Detailed descriptions for preparing chloroperoxidase can be found in the following articles and patent: (1) U.S. Patent No. 4,247,641, Neidleman et al, 1981; (2) Morris et al, *J. Biol. Chem. 241*, 1763, (1966); and (3) Cooney et al, *Biotech. Bioeng. 16*, 1045, (1974).

The haloperoxidase may also be used in an immobilized form. Processes for enzyme immobilization are familiar to those skilled in the art, and include reacting either a solution of the enzyme or a suspension of enzyme containing cells with one of a broad range of organic or inorganic supports. Included among these are polyacrylamide, ethylene-maleic acid copolymers, methacrylic-based polymers, polypeptides,

styrene-based polymers, agarose, cellulose, dextran, porous glass beads, and aluminum or titanium hydroxide. Enzymes in this form have increased stability, extended life and usefulness, and recoverability. Reactions employing immobilized enzymes may be run in columns or reaction tanks.

In addition to the haloperoxidase, an oxidizing agent is required in the reaction mixture. A preferred oxidizing agent is hydrogen peroxide, which may be added directly to the mixture in a single batch addition, or in a continuous slow feed. It may alternatively be generated as a slow feed *in situ* by the use of a hydrogen peroxide-producing enzyme system. Such enzyme systems are well known in the art, and include glucose-1-oxidase in the presence of D-glucose, pyranose-2-oxidase or glucose-2-oxidase in the presence of D-glucose, D- and L-amino acid oxidases in the presence of D- and L-methionine, methanol oxidase in the presence of methanol, and diamine oxidases in the presence of histamine. The hydrogen peroxide-generating system may be present in the non-immobilized or immobilized state as with the haloperoxidase. The hydrogen peroxide may also be generated by chemical reaction, such as by anthraquinone or isopropyl alcohol oxidation processes.

The hydrogen peroxide is present preferably in molar ratio of from about 0.5:1 to about 50:1, most preferably in a ratio of about 1:1 or less with respect to the alkene or alkyne. The molar ratio preferences refer to the average presence of hydrogen peroxide during the reaction. The actual molar ratio will usually vary during the reaction and the molar ratio at any particular time may be above or below the ranges cited. Other suitable oxidizing agents include organic peroxides, such as methyl, ethyl, or butyl peroxides.

Also, a source of two different water-soluble halide salts is required in the reaction mixture. The preferred halide salts are the fluoride, chloride, bromide and iodide salts of the alkali metals, sodium, potassium and lithium. Seawater or other natural brines which contain molar amounts of halide salts can be used. The total halide ion is present at a concentration greater than 2000 mM. The proportion of each halide ion ($X^-$ and $X_1^-$) in the reaction mixture is as follows:

$$[X_1^-] \gg [X^-]$$

$X^-$ is preferably present at a concentration of about the stoichiometric amount required for incorporation into product (mM amounts). $X_1^-$ is preferably in major excess (M amounts). The halide ion that is not directly incorporated by haloperoxidase or that has the higher oxidation potential of the two halide ions, is assigned to be $X_1^-$. The halide ion that is directly incorporated by haloperoxidase or that has the lower oxidation potential of the two halide ions, is assigned to be $X^-$.

The reaction is usually conducted with the pH range of from about 2.2 to about 8.0. The pH of the reaction may be maintained within the desired range by use of a buffering agent. Suitable buffers include sodium or potassium phosphate, gluconate, citrate, formate, and acetate based systems. Other suitable techniques besides buffering may be used for pH control and adjustment.

The reaction may be conducted in an aqueous medium. While some of the alkenes and alkynes that can be converted by the process are substantially insoluble in an aqueous medium, the reaction, nevertheless, proceeds satisfactorily under conditions of mixing, or other modes of dispersion, which provide sufficient substrate solubility for the reaction.

The reaction can also be conducted in the presence of aqueous organic solvent mixtures, such as water solutions containing lower aliphatic alcohols, dioxane, dimethylformamide, dimethylsulfoxide or glycerol, in order to increase substrate solubility.

The reaction is preferably conducted under aerobic conditions and in the temperature range of 15° to about 50°, preferably at 20° to about 30°.

As previously indicated, the components of the reaction mixture, namely the alkene or alkyne, the haloperoxidase, the oxidizing agent, the two different halide ions, and the buffering agent, are simply mixed together in water or mixed aqueous or organic media, and agitated for a period of from about 30 seconds to about 1 hour to obtain the vicinally, heterogeneously dihalogenated products.

The reaction for alkenes is represented by the following equation:

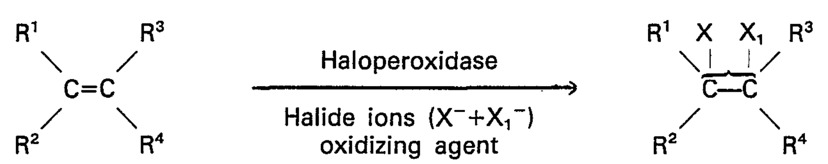


The reaction for alkynes is represented by the following equation:

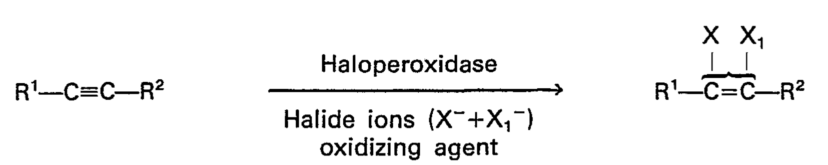


The products were quantitated by gas chromatography (GC) using flame ionization detection (FID). 5 µl

of the reaction mixture was injected into a Varian 3700 GC, equipped with a 6 foot×4 mm coiled, glass column packed with Tenax-GC (80/100 mesh). Flow rate through the column was set at 40 ml/minute of helium. The column temperature was operated isothermally (specific temperature given in each example); the injection temperature was set at 240°C; and the detector temperature was set at 240°C.

The products were identified by gas chromatography-mass spectrometry (GCMS). 10 μl of the reaction mixture was injected into a Finnigan 4021 GCMS, equipped with a 6 foot×4 mm coiled, glass column packed with Tenax-GC (80/100 mesh). Flow rate through the column was set at 30 ml/minute of helium. The column temperature was operated isothermally (specific temperature given in each example); the injection temperature was set at 240°C; and the jet separator was set at 240°C. The mass spectrometer was operated at 70 eV, electron impact ionization.

Resolution and measurement of certain isomeric, vicinally, heterogeneously dihalogenated products was obtained by capillary gas chromatography-mass spectrometry ($GC^2MS$). The concentrate of the ethyl ether extract of the reaction mixture was injected (10 μl) into a Finnigan 4021 GCMS, equipped with a 25 m×0.25 mm I.D. fused silica capillary column coated with SE-54 (purchased from J and W Scientific Company). Flow rate through the column was set at 2 ml/minute of helium. Samples were injected at 200°C with a 200:1 split injection. The column temperature was programmed from 60°C to 200°C at 5°C/minute. The mass spectrometer was operated at 70 eV, electron impact ionization. The capillary column was directly inserted into the ion source.

The following Examples are intended only further to illustrate the invention and not to limit the same.

Example 1

This Example demonstrates the control of the ratio of heterogeneous dihalogenated product to homogeneous dihalogenated product by controlling the proportions of the two different halide salts in the reaction. Bromide ion and chloride ion can each be utilized individually by chloroperoxidase.

Potassium bromide, potassium chloride, potassium phosphate buffer at pH 3.5 (10 ml, 0.3 M), hydrogen peroxide (4.1 mg; 137 μl of a 3% solution; 12 mM final) and allyl alcohol (7.0 mg; 12 mM final; $HOCH_2CH{=}CH_2$; purchased from Aldrich Chemical Company, Milwaukee, WI) were mixed together in a 25 ml Pyrex flask at room temperature and room pressure. The haloperoxidase enzyme, chloroperoxidase (0.4 ml) was then added. The reaction was concluded 15 minutes after the addition of the last reagent.

The following levels of bromide ion and chloride ion were used:

| Rxn | mM KBr final | mM KCl final | mM Total halide ion |
|---|---|---|---|
| A | 3000 | 0 | 3000 |
| B | 1200 | 1200 | 2400 |
| C | 20 | 2000 | 2020 |
| D | 4 | 2000 | 2004 |
| E | 0 | 2000 | 2000 |

The chloroperoxidase was prepared as follows:

Mycelial pads of *Caldariomyces fumago* (ATCC 16373) were grown on potato agar slants. Sliced potato (200 g) was cooked in distilled water (500 ml) for 40 minutes and then strained. A solution of glucose (21 g) and agar (20 g) in distilled water (500 ml) was added to the strained solution. The pH was adjusted to 6.8 and the volume was brought to 1 liter with distilled water. The medium was sterilized at 121°C for 15 minutes.

The organism was inoculated on the potato agar slants, produced in accordance with the above procedure, and was grown for about one week at room temperature. The organism was then used to inoculate a soybean-glucose medium (50 ml). The soybean-glucose medium was prepared by adding, to 1 liter of distilled, extraction process soybean meal (30 g), glucose (30 g), and $CaCO_3$ (7 g). The medium was sterilized at 121°C for 30 minutes and was then inoculated with the organism after cooling.

The organism was grown for 4—5 days on a rotary shaker at 25°C. 5 ml of this material was used to inoculate a 500 ml Erlenmeyer flask containing 100 ml of a modified Czapek-Dox medium prepared by adding the following to 1 liter of distilled water: $NaNO_3$ (3 g), $KH_2PO_4$ (1 g), KCl (0.5 g), $MgSO_4 \cdot 7H_2O$ (0.5 g), $FeSO_4 \cdot 7H_2O$ (10 mg), and glucose (40 g). The medium was sterilized at 121°C for 20 minutes prior to inoculation with the organism.

The organism was grown under static conditions at room temperature for 5—7 days. The black mycelial pads which formed were collected, rinsed with distilled water, and stored in plastic bags in a freezer at −10°C for subsequent use.

The haloperoxidase was prepared by grinding 6 mycelial pads (prepared in accordance with the above procedures) with 60 g acid-washed sand and 60 ml distilled water for 2 minutes in a Virtis 45 homogenizer.

The homogenate was centrifuged while cold and the supernatant solution was used as the source of the haloperoxidase, chloroperoxidase.

The final chloroperoxidase supernatant was filtered through Whatman® No. 1 paper at room temperature. The filtrate was concentrated about 10-fold using a rotary film evaporator at reduced pressure and temperature (<35°C). The concentrate was chilled at 0°C in an ice bath, and prechilled (0°) ethanol was added until 45% ethanol (v/v) was reached. The mixture was stirred vigorously for 15 minutes, and then centrifuged at −10°C (at 15,000 g) with a 55—34 rotor in a Sorval RC-5 Superspeed for 15 minutes. The black sediment was discarded. To the centrifugate, cooled at 0°C, was added additional prechilled ethanol to give 65% ethanol (v/v). The mixture was slowly stirred for 30 minutes at 0°C, and then centrifuged as before. The centrifugate was discarded, and the precipitate containing the chloroperoxidase activity was dissolved in 1 ml of 0.05 M potassium phosphate buffer (pH 7). The enzyme solution was stored at −20°C.

The products were quantitated by gas chromatography (GC) using flame ionization detection (FID). 5 µl of the reaction mixture was injected into a Varian 3700 GC, equipped with a 6 foot×4 mm coiled, glass column packed with Tenax-GC® (80/100 mesh). Flow rate through the column was set at 40 ml/minute of helium. The column temperature was set at 190°C, isothermal; the injection temperature was set at 190°C, isothermal; the injection temperature was set at 240°C; and the detector temperature was set at 240°C.

The products were identified by gas chromatography-mass spectrometry (GCMS). 10 µl of the reaction mixture was injected into a Finnigan 4021 GCMS, equipped with a 6 foot×4 mm coiled, glass column packed with Tenax-GC® (80/100 mesh). Flow rate through the column was set at 30 ml/minute of helium. The column temperature was set at 190°C, isothermal; the injection temperature was set at 240°C; and the jet separator was set at 240°C; the mass spectrometer was operated at 70 eV, electron impact ionization.

Seven products were detected.

One product had a GC retention time of 6 minutes and showed the mass spectrum diagnostic for 2,3-dichloro-1-propanol: molecular mass ion not detected; major fragment mass ions at mass 92 and 94 (3:1 in intensity; loss of HCl from molecular ion), and at mass 62 and 64 (3:1 in intensity; the $CH_2CHCl^+$ ion). This product had an identical GC retention time and mass spectrum with that of an authentic sample of 2,3-dichloro-1-propanol (purchased from Aldrich Chemical Company).

Two other products had GC retention times of 7 and 8 minutes, and showed the mass spectra diagnostic for chloropropanediols. The product having an 7 minute retention time was identified as 1-chloro-2,3-propanediol: molecular mass ion not detected; major fragment mass ions at mass 79 and 81 (3:1 in intensity; loss of $CH_2OH$ from the molecular ion), and at mass 61 (loss of $CH_2Cl$ from molecular ion). The product having an 8 minute retention time was identified as 2-chloro-1,3-propanediol: molecular mass ion not detected; major fragment mass ions at mass 92 and 94 (3:1 in intensity; loss of $H_2O$ from molecular ion), and at mass 62 and 64 (3:1 in intensity; the $CH_2CHCl^+$ ion).

Another product had a GC retention time of 10 minutes, and showed the mass spectrum diagnostic for bromochloro-1-propanol: molecular mass ion at mass 172, 174 and 176 (3:4:1 in intensity) indicating one chlorine atom and one bromine atom on the molecule; major fragment mass ions at mass 136 and 138 (1:1 in intensity; loss of HCl from molecular ion), at mass 106 and 108 (1:1 in intensity; the $CHCH_2Br^+$ ion), and at mass 92 and 94 (3:1 in intensity; loss of HBr from molecular ion).

Two other products had GC retention time of 11 and 12 minutes, and showed the mass spectra diagnostic for bromo-propanediols. The product having an 11 minute retention time was identified as 1-bromo-2,3-propanediol: molecular ion not detected; major fragment mass ions at mass 123 and 125 (1:1 in intensity; loss of $CH_2OH$ from molecular ion), and at mass 61 (loss of $CH_2Br$ from molecular ion). The product having a 12 minute retention time was identified as 2-bromo-1,3-propanediol: molecular mass ion not detected; major fragment mass ions at mass 136 and 138 (1:1 in intensity; loss of $H_2O$ from molecular ion), and at mass .106 and 108 (1:1 in intensity; the $CH_2CHBr^+$ ion).

A final product had a GC retention time of 16 minutes and showed the mass spectrum diagnostic for 2,3-dibromo-1-propanol: molecular mass ion at mass 216, 218 and 220 (1:2:1 in intensity) indicating 2 bromine atoms on the molecule; major fragment mass ions at 137 and 139, and 136 and 138 (both sets 1:1 in intensity; loss of Br and HBr, respectively, from molecular ion) and at mass 106 and 108 (1:1 in intensity; the $CH_2CHBr^+$ ion). This product had an identical GC retention time and mass spectrum with that of an authentic samples of 2,3-dibromo-1-propanol (purchased from Aldrich Chemical Company).

The following summarizes the products obtained:

| Product | % of Total yield | | | | |
|---|---|---|---|---|---|
| | Rxn A | Rxn B | Rxn C | Rxn D | Rxn E |
| 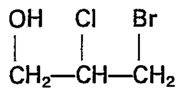 | 0 | 69 | 95 | 73 | 0 |
| 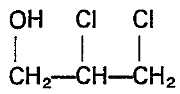 | 0 | 0 | 0 | 24 | 91 |
| 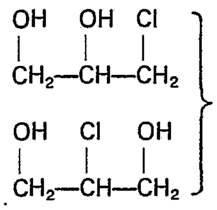 | 0 | 0 | 0 | 2 | 9 |
| 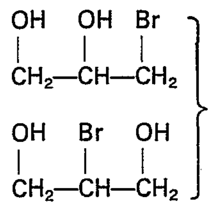 | 1 | 1 | 0 | 0 | 0 |
| 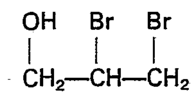 | 99 | 31 | 5 | 1 | 0 |
| Total yield | 21.7 mg | 15.2 mg | 13.9 mg | 9.7 mg | 5.4 mg |

Reaction C thus demonstrates the proportions of the two halide ion levels needed for this heterogeneous, dihalogenated product reaction.

Further analysis of the bromochloro-1-propanol product was made using capillary gas chromatography-mass spectrometry ($GC^2MS$). The reaction mixture was extracted with ethyl ether, and this extract when concentrated was injected (10 µl) into a Finnigan 4021 GCMS, equipped with a 25 m×0.25 mm I.D. fused silica capillary column coated SE-54. Flow rate through the column was set at 2 ml/minute of helium. Sample was injected at 200°C with a 200:1 split injection. The column temperature was programmed from 60°C to 150°C at 5°C/minute. The mass spectrometer was operated at 70 eV, electron impact ionization. The capillary column was directly inserted into the ion source.

2-Bromo-3-chloro-1-propanol had a $GC^2$ retention time of 3.6 minutes, and showed the following diagnostic mass spectrum: molecular mass ion not detected; major fragment mass ions at mass 136 and 138 (1:1 in intensity; loss of HCl from molecular ion), mass 106 and 108 (1:1 in intensity; the $CH_2CHBr^+$ ion), and at mass 57 (loss of HBrCl from molecular ion).

3-Bromo-2-chloro-1-propanol had a $GC^2$ retention time of 3.8 minutes, and showed the following diagnostic mass spectrum: molecular mass ion at mass 172, 174 and 176 (3:4:1 in intensity) indicating one chlorine atom and one bromine atom on the molecule; major fragment mass ions at mass 93 and 95 (3:1 in intensity; loss of Br from molecular ion), at mass 92 and 94 (3:1 in intensity; loss of HBr from molecular ion), and at mass 57 (loss of HBrCl from molecular ion).

The ratio of product formed was 1:1.2; 2-bromo-3-chloro-1-propanol: 3-bromo-2-chloro-1-propanol.

In addition, the bromochloro-1-propanol products were converted to epichlorohydrin and epibromohydrin by addition of lime to the aqueous reaction mixture until the pH was greater than 10. Identity of epichlorohydrin and epibromohydrin was confirmed by gas chromatography-mass spectrometry comparison with authentic samples (purchased from Aldrich Chemical Company).

Further, the bromochloro-1-propanol products were converted to epichlorohydrin and epibromohydrin by addition of *Flavobacterium* sp. whole cells to the aqueous reaction mixture, as set forth in U.S. Patent No. 4,247,641 (Neidleman et al, 1981).

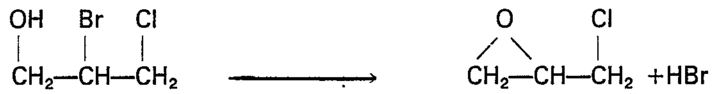

2-bromo-3-chloro-1-propanol — epichlorohydrin

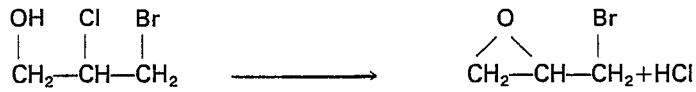

3-bromo-2-chloro-1-propanol — epibromohydrin

Example 2

This example demonstrates control of the ratio of heterogeneous dihalogenated product to homogeneous dihalogenated product by adjusting the proportions of the two different halide salts in the reaction. Bromide ion can be utilized by lactoperoxidase individually but chloride ion cannot.

The procedure of Example 1 was followed, except 1) sodium chloride was substituted for potassium chloride, 2) lactoperoxidase (purchased from Sigma Chemical Company, St. Louis, MO; Catalog No. L-7129; 0.5 ml used per reaction) was substituted for chloroperoxidase, and 3) the buffer pH was set at 6.5 rather than 3.5.

The following levels of bromine ion and chloride ion were used:

| Rxn | mM KBr final | mM NaCl final | mM Total halide ion |
|---|---|---|---|
| A | 3000 | 0 | 3000 |
| B | 1200 | 1200 | 2400 |
| C | 20 | 2000 | 2020 |
| D | 4 | 2000 | 2004 |
| E | 0 | 2000 | 2000 |

Analysis of products by GC and GCMS was as outlined in Example 1.

The following summarizes the products obtained as a function of halide concentrations in the reaction mixture:

| | % of Total yield | | | | |
|---|---|---|---|---|---|
| Product | Rxn A | Rxn B | Rxn C | Rxn D | Rxn E |
| 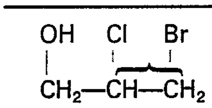 | 0 | 61 | 93 | 98 | 0 |
| 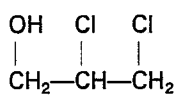 | 0 | 0 | 0 | 0 | 0 |
| 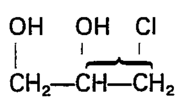 | 0 | 0 | 0 | 0 | 0 |
| 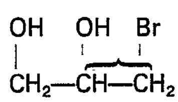 | 1 | 0 | 0 | 0 | 0 |
| 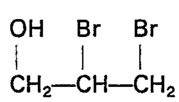 | 98 | 49 | 7 | 2 | 0 |
| Total yield | 20.8 mg | 18.7 mg | 14.0 mg | 6.2 mg | 0 mg |

Reactions C and D thus demonstrate the proportions of the two halide ion levels needed for this heterogeneous, dihalogenated product reaction.

Example 3

The procedure of Example 1 was followed, except potassium iodide was substituted for potassium bromide. Chloride ion and iodide ion can each be utilized individually by chloroperoxidase.

The following level of chloride ion and iodide ion was used:

| mM KI final | mM KCl final | mM Total halide ion |
|---|---|---|
| 20 | 2000 | 2020 |

Analysis of products by GC and GCMS was as outlined in Example 1. The GC column temperature was set at 210°C, isothermal. Seven products were detected.

One product had a GC retention time of 28 minutes and showed the mass spectrum diagnostic for 2,3-diiodo-1-propanol: molecular mass ion not detected; major fragment mass ions at mass 254 (the $I_2^+$ ion), at mass 185 (loss of I from molecular ion), at mass 127 (the $I^+$ ion), and at mass 57 (loss $HI_2$ from molecular ion).

Two other products had GC retention times of 9 and 11 minutes, and showed the mass spectra diagnostic for iodopropanediols. The products having a 9 minute retention time was identified as 1-iodo-2,3-propanediol: molecular mass ion at mass 202; major fragment mass ions at mass 171 (loss of $CH_2OH$ from molecular ion) and at mass 75 (loss of I from molecular ion). The product having a 11 minute retention time was identified as 2-iodo-1,3-propanediol: molecular mass ion at mass 202; major fragment mass ions at mass 154 (loss of $CH_2OH$+OH from molecular ion) at mass 127 (the $I^+$ ion) and at mass 75 (loss of I from molecular ion).

Another product had a GC retention time of 7 minutes, and showed the mass spectrum diagnostic for chloroiodo-1-propanol: molecular mass ion at mass 220 and 222 (3:1 in intensity) indicating one chlorine atom on the molecule; major fragment mass ions at mass 127 (the $I^+$ ion), at mass 93 and 95 (3:1 in intensity; loss of I from molecular ion), and at mass 57 (loss of HClI from molecular ion).

Another product had a GC retention time of 2 minutes and showed the mass spectrum diagnostic for 2,3-dichloro-1-propanol: molecular mass ion not detected; major fragment mass ions at mass 92 and 94 (3:1 in intensity; loss of HCl from molecular ion), and at mass 62 and 64 (3:1 in intensity; the $CH_2CHCl^+$ ion). This product had an identical GC retention time and mass spectrum with that of an authentic sample of 2,3-dichloro-1-propanol (purchased from Aldrich Chemical Company).

And two other products had GC retention times of 3 and 4 minutes, and showed the mass spectra diagnostic for chloropropanediols. The product having a 3 minute retention time was identified as 1-chloro-2,3-propanediol: molecular mass ion not detected; major fragment mass ions at mass 79 and 81 (3:1 in intensity; loss of $CH_2OH$ from the molecular ion), and at mass 61 (loss of $CH_2Cl$ from molecular ion). The product having a 4 minute retention time was identified as 2-chloro-1,3-propanediol: molecular mass ion not detected; major fragment mass ions at mass 92 and 94 (3:1 in intensity; loss of $H_2O$ from molecular ion), and at mass 62 and 64 (3:1 in intensity; the $CH_2CHCl^+$ ion).

Molecular iodine ($I_2$) was also formed.

The following summarizes the products obtained:

| Product | % of Total yield |
|---|---|
| 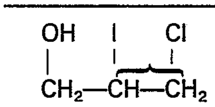 | 46 |
| 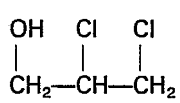 | 17 |
| 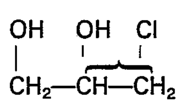 | 8 |
| 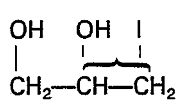 | 21 |
| 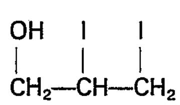 | 8 |

Total yield=5.3 mg

Further analysis of the chloroiodo-1-propanol product was made using capillary gas chromatography-mass spectrometry ($GC^2MS$) as described in Example 1.

2-Chloro-3-iodo-1-propanol had a $GC^2$ retention time of 6.4 minutes, and showed the following diagnostic mass spectrum: molecular mass ion at mass 220 and 222 (3:1 in intensity) indicating one chlorine atom on the molecular; major fragment mass ions at mass 127 (the $I^+$ ion), at mass 93 and 95 (3:1 in intensity; loss of I from molecular ion), and at mass 57 (loss of HClI from molecular ion).

3-Chloro-2-iodo-1-propanol had a $GC^2$ retention time of 6.2 minutes, and showed the following diagnostic mass spectrum: molecular mass ion at mass 220 and 222 (3:1 in intensity) indicating one chlorine atom on the molecule; major fragment mass ions at mass 93 and 95 (3:1 in intensity; loss of I from molecular ion), and at mass 57 (loss of HClI from molecular ion).

The ratio of product formed was 1:1; 2-chloro-3-iodo-1-propanol: 3-chloro-2-iodo-1-propanol.

In addition, the chloroiodo-1-propanols were converted to epichlorohydrin and epiiodohydrin by addition of lime to the aqueous reaction mixture until the pH was greater than 10. Identity of epichlorohydrin and epiiodohydrin was confirmed by gas chromatography-mass spectrometry comparison with authentic samples (purchased from Aldrich Chemical Company).

Further, the chloroiodo-1-propanols were converted to epichlorohydrin and epiiodohydrin by addition of *Flavobacterium* sp. whole cells to the aqueous reaction mixture, as set forth in U.S. Patent No. 4,247,641 (Neidleman et al, 1981).

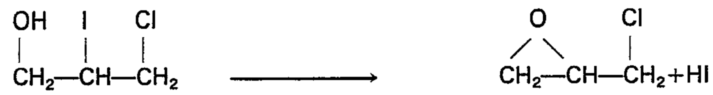

3-chloro-2-iodo-propanol | epichlorohydrin

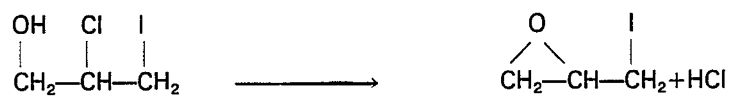

2-chloro-3-iodo-1-propanol | epiiodohydrin

Example 4

The procedure of Example 1 was followed, except 1) potassium iodide and sodium fluoride were substituted for potassium bromide and potassium chloride, 2) horseradish peroxide (2 mg; purchased from Sigma Chemical Company, Catalog No. P-8375) was substituted for chloroperoxidase, and 3) the buffer pH was set at 6.5 rather than 3.5. Iodine ion can be utilized by horseradish peroxidase individually but fluoride ion cannot.

The following level of iodide ion and fluoride ion was used:

| mM KI final | mM NaF final | mM Total halide ion |
|---|---|---|
| 100 | 2000 | 2100 |

Analysis of products by GC and GCMS was as outlined in Example 1. The GC column temperature was set at 210°C, isothermal. Five products were detected.

One product had a GC retention time of 28 minutes and showed the mass spectrum diagnostic for 2,3-diiodo-1-propanol: molecular mass ion not detected; major fragment mass ions at mass 254 (the $I_2^+$ ion), at mass 185 (loss of I from molecular ion), at mass 127 ($I^+$ ion), and at mass 57 (loss of $HI_2$ from molecular ion).

Two other products had GC retention times of 9 and 11 minutes, and showed the mass spectra diagnostic for iodopropanediols. The product having a 9 minute retention time was identified as 1-iodo-2,3-propanediol: molecular mass ion at mass 202; major fragment mass ions at mass 171 (loss of $CH_2OH$ from molecular ion) and at mass 75 (loss of I from molecular ion). The product having a 11 minute retention time was identified as 2-iodo-1,3-propanediol: molecular mass ion at mass 202; major fragment mass ions at mass 154 (loss of $CH_2OH+OH$ from molecular ion) at mass 127 (the $I^+$ ion) and at mass 75 (loss of I from molecular ion).

Another product had a GC retention time of 5 minutes, and showed the mass spectrum diagnostic for 2-fluoro-3-iodo-1-propanol: molecular mass ion at mass 204; major fragment mass ions at mass 127 (the $I^+$ ion), and at mass 77 (loss of I from molecular ion).

And another product had a GC retention time of 4 minutes, and showed the diagnostic mass spectrum for 3-fluoro-2-iodo-1-propanol: molecular mass ion at mass 204; major fragment mass ions at mass 154 (the $CH_2CHI^+$ ion) at mass 127 (the $I^+$ ion), and at mass 77 (loss of I from molecular ion).

Molecular iodine ($I_2$) was also formed.

The following summarizes the products obtained:

| Product | % of Total yield |
|---|---|
| 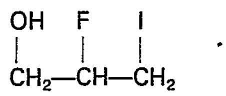 | 8 |
| 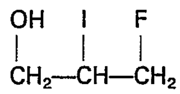 | 9 |
| 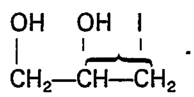 | 52 |
| 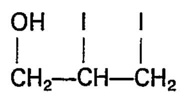 | 31 |

Total yield=4.3 mg

This procedure thus provides a method for the production of 2-fluoro-3-iodo-1-propanol and 3-fluoro-2-iodo-1-propanol, both of which are new compositions of matter. 3-Fluoro-2-iodo-1-propanol may be treated with a base such as lime or caustic soda, as shown below, to form epifluorohydrin, an epoxide having known utility in the polymer industry. 2-Fluoro-3-iodo-1-propanol may be converted by catalytic hydrogenation, as shown below, to form propylenefluorohydrin, the structure of which indicates utility as an intermediate for producing fluorinated esters useful in the polymer industry.

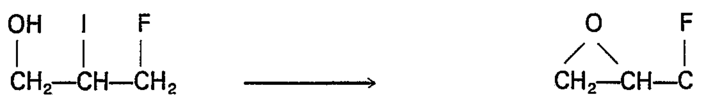

3-fluoro-2-iodo-1-propanol epifluorohydrin

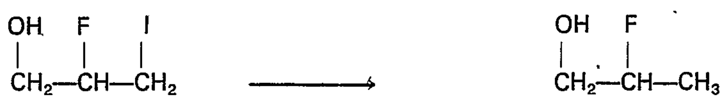

2-fluoro-3-iodo-1-propanol propylene fluorohydrin

Example 5

The procedure of Example 2 was followed, except: 1) the final potassium bromide and sodium chloride levels were 20 mM and 2000 mM, respectively, and 2) *in situ* generation of hydrogen peroxide was made by adding β-D-glucose (25 mg) and glucose-1-oxidase (0.05 ml; purchased from Sigma Chemical Company, Catalog No. G-6500). The reaction was allowed to proceed for 2 hours.

The following summarizes the products obtained:

| Product | % of Total yield |
|---|---|
| 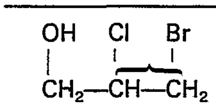 | 91 |
| 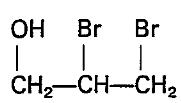 | 9 |

Total yield=8.6 mg

Example 6

The procedure of Example 1 was followed, except 2-butene-1,4-diol (11 mg; 12 mM final; $HOCH_2CH=CHCH_2OH$; purchased from Aldrich Chemical Company) was substituted for allyl alcohol.

The following level of bromide ion and chloride ion was used:

| mM KBr | mM KCl | mM Total halide ion |
|---|---|---|
| 20 | 2000 | 2020 |

Analysis of products by GC and GCMS was as outlined in Example 1. The GC column temperature was set at 210°C, isothermal. Two products were detected.

One product had a GC retention time of 19 minutes and showed the mass spectrum diagnostic for 2,3-dibromo-1,4-butanediol: molecular mass ion not detected; major fragment mass ions at mass 198, 200 and 202 (1:2:1 in intensity; loss of $CH_2OH$+OH from molecular ion), mass 167 and 169 (1:1 in intensity; loss of Br from molecular ion), and at mass 149 and 151 (1:1 in intensity; loss of Br+$H_2O$ from molecular ion).

The other product had a GC retention time of 15 minutes and showed the mass spectrum diagnostic for 2-bromo-3-chloro-1,4-butanediol: molecular mass ion at mass 202, 204 and 206 (3:4:1 in intensity) indicating one bromine atom and one chlorine atom on the molecule; major fragment mass ions at mass 154, 156 and 158 (3:4:1 in intensity; loss of $CH_2OH$+OH from molecular ion), at mass 167 and 169 (1:1 in intensity; loss of Cl from molecular ion), at mass 123 and 125 (3:1 in intensity; loss of Br from molecular ion), and at mass 105 and 107 (3:1 in intensity; loss of Br+$H_2O$ from molecular ion).

The following summarizes the products obtained:

| Product | % of Total yield |
|---|---|
| 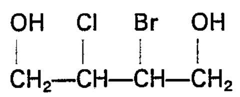 | 83 |
| 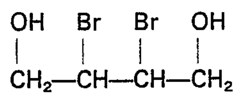 | 17 |

Total yield=10.6 mg

This procedure thus provides a method for the production of 2-bromo-3-chloro-1,4-butanediol, a new composition of matter. As shown below, 2-bromo-3-chloro-1,4-butanediol may be reacted over an extended time with a base such as lime or caustic soda to form butadiene diepoxide, an epoxide having known utility in the polymer industry.

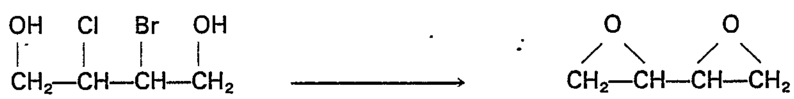

2-bromo-3-chloro-1,4-butanediol butadiene diepoxide

Example 7

The procedure of Example 6 was followed except 2-butyne-1,4-diol (10.3 mg; 12 mM final; $HOCH_2C{\equiv}CCH_2OH$; purchased from Aldrich Chemical Company) was substituted for 2-butene-1,4-diol.

Two products were detected. The GC column temperature was set at 210°C, isothermal.

One product had a GC retention time of 13 minutes, and showed the mass spectrum diagnostic for 2-bromo-3-chloro-2-butene-1,4-diol: molecular ion at mass 200, 202 and 204 (3:4:1 in intensity) indicating one bromine atom and one chlorine atom on the molecule; major fragment mass ions at mass 182, 184 and 186 (3:4:1 in intensity; loss of $H_2O$ from molecular ion), at mass 165 and 167 (1:1 in intensity; loss of Cl from molecular ion), and at mass 147 and 149 (1:1 in intensity; loss of Cl+$H_2O$ from molecular ion).

And the other product had a GC retention time of 17 minutes, and showed the mass spectrum diagnostic for 2,3-dibromo-2-butene-1,4-diol: molecular mass ion at mass 244, 246 and 248 (1:2:1 in intensity) indicating two bromine atoms on the molecule; major fragment mass ions at mass 226, 228, 230 (1:2:1 in intensity; loss of $H_2O$ from molecular ion), at mass 165 and 167 (1:1 in intensity; loss of Br from molecular ion), at mass 147 and 149 (1:1 in intensity; loss of Br+$H_2O$ from molecular ion).

The following summarizes the products obtained:

| Product | % of Total yield |
|---|---|
| 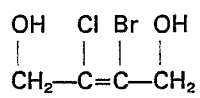 | 86 |
| 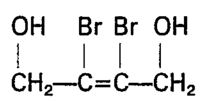 | 14 |

Total yield=6.9 mg

Example 8

The procedure of Example 6 was followed, except ethylene was substituted for 2-butene-1,4-diol.

Ethylene ($CH_2=CH_2$; purchased from Matheson Gas Products, Lyndhurst, NJ), a gaseous alkene, was slowly (10 ml/min) and continuously bubbled through the reaction mixture. After 15 minutes, the haloperoxidase enzyme chloroperoxidase (0.4 ml), was then added. Finally hydrogen peroxide (4.1 mg; 12 mM final) was added. The reaction was concluded 15 minutes after the addition of the last reagent.

Five products were detected. The GC column temperature was set at 170°C, isothermal.

One product had a GC retention time of 7 minutes and showed the mass spectrum diagnostic for 1,2-dibromo ethane: molecular mass ion at mass 186, 188 and 190 (1:2:1 in intensity) indicating two bromine atoms on the molecule; major fragment mass ions at 107 and 109 (1:1 in intensity; loss of Br from molecular ion), and at mass 93 and 95 (1:1 in intensity; the $CH_2Br^+$ ion). This product had an identical GC retention time and mass spectrum with that of an authentic sample of 1,2-dibromo ethane (purchased from Aldrich Chemical Company).

Another product had a GC retention time of 5 minutes and showed the mass spectrum diagnostic for 2-bromo ethanol; molecular mass ion at mass 124 and 126 (1:1 in intensity) indicating one bromine atom on the molecule; major fragment mass ions at mass 123 and 125 (1:1 in intensity; loss of H from molecular ion) and at mass 93 and 95 (1:1 in intensity; the $CH_2Br^+$ ion).

A third product had a GC retention time of 4 minutes and showed the mass spectrum diagnostic for 1-bromo-2-chloro ethane: molecular mass ion at mass 142, 144 and 146 (3:4:1 in intensity) indicating one bromine atom and one chlorine atom on the molecule; major fragment mass ions at mass 93 and 95 (1:1 in intensity; the $CH_2Br^+$ ion), at mass 79 and 81 (1:1 in intensity; the $Br^+$ ion) and at mass 63 and 65 (3:1 in intensity; loss of Br from molecular ion).

Another product had a GC retention time of 2 minutes and showed the mass spectrum diagnostic for 1,2-dichloro ethane: molecular mass ion at mass 98, 100 and 102 (10:6:1 in intensity) indicating two chlorine atoms on the molecule; major fragment mass ions at mass 62 and 64 (3:1 in intensity; loss of HCl from molecular ion), and at mass 49 and 51 (3:1 in intensity; the $CH_2Cl^+$ ion). This product had an identical GC retention time and mass spectrum with that of an authentic sample of 1,2-dichloro ethane (purchased from Aldrich Chemical Company).

And the other product had a GC retention time of 3 minutes and showed the mass spectrum diagnostic for 2-chloro ethanol: molecular mass ion at mass 80 and 82 (3:1 in intensity) indicating one chlorine atom on the molecule; major fragment mass ions at mass 49 and 51 (3:1 in intensity; the $CH_2Cl^+$ ion), and at mass 44 (loss of HCl from molecular ion).

The following summarizes the products obtained:

| Product | % of Total yield |
|---|---|
| 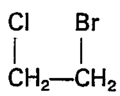 | 28 |
| 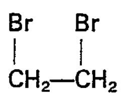 | 19 |
| 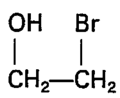 | 22 |
| 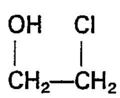 | 14 |
| 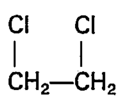 | 17 |

Total yield=6.5 mg

Example 9

The procedure of Example 8 was followed, except 1) lactoperoxidase (0.5 ml) was substituted for chloroperoxidase, 2) potassium iodide was substituted for potassium bromide, and 3) the buffer pH was set at 6.5 rather than 3.5.

The following level of chloride ion and iodide ion was used:

| mM KI final | mM KCl final | mM Total halide ion |
|---|---|---|
| 20 | 2000 | 2020 |

Three products were detected. The GC column temperature was set at 180°C, isothermal.

One product had a GC retention time of 9 minutes and showed the mass spectrum diagnostic for 1-chloro-2-iodo ethane: molecular mass ion at mass 190 and 192 (3:1 in intensity) indicating one chlorine atom on the molecule; major fragment mass ions at mass 155 (loss of Cl from molecular ion), at mass 127 (the $I^+$ ion), and at mass 63 and 65 (3:1 in intensity; loss of I from molecular ion).

Another product had a GC retention time of 15 minutes and showed the mass spectrum diagnostic for 1,2-diiodo ethane: molecular mass ion at mass 282; major fragment mass ions at mass 254 (the $I_2^+$ ion), at mass 155 (loss of I from molecular ion), and at mass 127 (the $I^+$ ion).

And the other product had a GC retention time of 10 minutes and showed the mass spectrum diagnostic for 2-iodo ethanol: molecular mass ion at mass 172; major fragment mass ions at mass 142 (the $CH_2I^+$ ion), and at mass 127 (the $I^+$ ion).

Molecular iodine was also detected.

The following summarizes the products obtained:

| Product | % of Total yield |
|---|---|
| 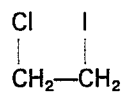 | 38 |
| 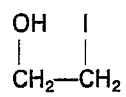 | 47 |
| 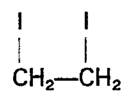 | 15 |

Total yield=4.3 mg

Example 10

The procedure of Example 2 was followed, except allyl chloride (9.1 mg, 12 mM final; $ClCH_2CH{=}CH_2$; purchased from Aldrich Chemical Company) was substituted for allyl alcohol.

The following level of bromide ion and chloride ion was used:

| mM KBr | mM KCl | mM Total halide ion |
|---|---|---|
| 20 | 2000 | 2020 |

Analysis of products by GC and GCMS was as outlined in Example 1. The GC column temperature was set at 190°C, isothermal. Four products were detected.

One product had a GC retention time of 8 minutes and showed the mass spectrum diagnostic for bromochloro-1-chloropropane: molecular mass ion at mass 190, 192 and 194 (6:10:5 in intensity) indicating 1 bromine atom and 2 chloride atoms on the molecule; major fragment mass ions at mass 155, 157 and 159 (3:4:1 in intensity; loss of Cl from molecular ion), at mass 141, 143 and 145 (3:4:1 in intensity; loss of $CH_2Cl$ from molecular ion), at mass 111, 113 and 115 (10:6:1 in intensity; loss of Br from molecular ion), at mass 93 and 95 (1:1 in intensity; the $CH_2Br^+$ ion), and at mass 49 and 51 (3:1 in intensity; the $CH_2Cl^+$ ion).

Another product had a GC retention time of 15 minutes and showed the mass spectrum diagnostic for 1,2-dibromo-3-chloropropane: molecular mass ion not detected; major fragment mass ions at mass 185, 187 and 189 (1:2:1 in intensity; loss of $CH_2Cl$ from molecular ion), at mass 155, 157 and 159 (3:4:1 in intensity; loss of Br from molecular ion), at mass 75 and 77 (3:1 in intensity; loss of $HBr_2$ from molecular ion), at mass 93 and 95 (1:1 in intensity; the $CH_2Br^+$ ion), and at mass 49 and 51 (3:1 in intensity; the $CH_2Cl^+$ ion).

The two other products had GC retention times of 9 and 11 minutes, respectively, and showed the mass spectra diagnostic for bromochloropropanols. The product having an 11 minute retention time was identified as 2-bromo-1-chloro-3-propanol: molecular mass ion at mass 172, 174 and 176 (3:4:1 in intensity) indicating the presence of one bromine atom and one chlorine atom on the molecule; major fragment mass ions at mass 136 and 138 (1:1 in intensity; loss of HCl from molecular ion), at mass 106 and 108 (1:1 in intensity; the $CH_2CH{-}Br^+$ ion), and at mass 49 and 51 (3:1 in intensity; the $CH_2Cl^+$ ion). The product having a 9 minute retention time was identified as 1-bromo-3-chloro-2-propanol: molecular mass ion at mass 172,

174 and 176 (3:4:1 in intensity) indicating the presence of one bromine atom and one chlorine atom on the molecule; major fragment mass ions at mass 123 and 125 (1:1 in intensity; loss of $CH_2Cl$ from molecular ion), and at mass 79 and 81 (3:1 in intensity; loss of $CH_2Br$ from molecular ion).

The following summarizes the products obtained:

| Product | % of Total yield |
| --- | --- |
| 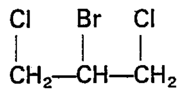 | 22 |
| 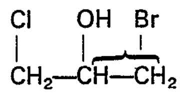 | 15 |
| 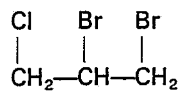 | 63 |

Total yield=10.1 mg

Analysis of products by GC and GCMS was as outlined in Example 1.

Various modifications in addition to those shown and described herein will become apparent to those skilled in the art from the foregoing description. Such modifications are intended to fall within the scope of the invention.

References cited

United States patents

| | | |
| --- | --- | --- |
| 3,576,893 | 4/1971 | Baird Jr., et. al. |
| 4,147,733 | 4/1979 | Fiske et. al. |
| 4,247,641 | 1/1981 | Neidleman et. al. |

Application No.

| | | |
| --- | --- | --- |
| 330,157 | 12/1981 | Geigert et. al.=US—A—4426449 1/1984 |

Other patents

Poland

105,589 1/1980 Dul et. al.

United Kingdom

2,028,173A filed Keller et. al.

(Application) 8/3/1979.

Other publications

1. Johnson, R. L. and D. W. Setser, *Journal of Physical Chemistry*, Vol. 71, 1967, pp. 4366—4383.
2. Roth, H. and H. R. Richardson, *Journal of Economic Entymology*, Vol. 63, 1970, pp. 496—499.
3. Rylander, P., *Catalytic Hydrogenation in Organic Synthesis,* Ch. 13, 1979, pp. 235—250.
4. Morrison, M. and G. R. Schonbaum, *Annual Reviews of Biochemistry*, Vol. 45, 1976, pp. 861—888.
5. Hager, L. P., D. R. Morris, F. S. Brown and H. Eberwein, *Journal of Biological Chemistry*, Vol. 241, 1966, pp. 1769—1777.
6. Bakkenist, A. R. J., J. E. G. DeBoer, J. Plat and R. Wever, *Biochimica et Biophysica Acta*, Vol. 613, 1980, pp. 337—348.
7. Morris, D. R. and L. P. Hager, *Journal of Biological Chemistry*, Vol. 241, 1966, pp. 1763—1768.
8. Cooney, C. L. and J. Hueter, *Biotechnology and Bioengineering*, Vol. 16, 1974, pp. 1045—1053.

**Claims**

1. A method for the vicinal, heterogeneous dihalogenation of alkenes and alkynes comprising reacting together a haloperoxidase, an oxidizing agent, two different halide ions which are both capable of being used by the haloperoxidase and the total concentration of both of which together is greater than 2000 mM, and an alkene or alkyne susceptible to dihalogenation by the haloperoxidase for a sufficient period of time to convert the alkene or alkyne to a vicinally heterogeneously dihalogenated product.

2. A method as claimed in claim 1, wherein the haloperoxidase is derived from the microorganism *Caldariomyces fumago* (ATCC 16373), algae, milk, thyroid, leukocytes or horseradish.

3. A method as claimed in claim 1 or claim 2, wherein the oxidizing agent is hydrogen peroxide.

4. A method as claimed in claim 3, wherein the hydrogen peroxide is present at a molar ratio to the alkene or alkyne of from about 0.5:1 to about 50:1.

5. A method as claimed in claim 3 or claim 4, wherein the hydrogen peroxide is generated *in situ*.

6. A method as claimed in any one of claims 1 to 5, wherein the halide ion sources are water soluble halide salts selected from the fluoride, chloride, bromide and iodide salts of sodium, potassium and lithium.

7. A method as claimed in any one of claims 1 to 5, wherein the halide ion sources are selected from seawater or other natural brines.

8. A method as claimed in any one of claims 1 to 7, wherein the reaction is conducted at a pH of from about 2.8 to about 8.0.

9. A method as claimed in any one of claims 1 to 8, wherein the two different halide ions consist of one halide ion present at a level at about the stoichiometric amount required for incorporation into the product, and the second halide ion present at a level in major excess to that required for incorporation into the product.

10. A method as claimed in any one of claims 1 to 9, wherein the reaction is effected in an aqueous environment at ambient conditions of temperature and pressure.

11. A method as claimed in any one of claims 1 to 10, wherein the alkene or alkyne is ethylene, allyl chloride, allyl alcohol, 2-butene-1,4-diol, or 2-butyne-1,4-diol.

12. A method as claimed in claim 1 wherein either the haloperoxidase is lactoperoxidase and the two different halide ions are chloride and bromide ions or chloride and iodide ions, or, the haloperoxidase is horseradish peroxidase and the two different halide ions are bromide and iodide ions or chloride and iodide ions or fluoride and iodide ions.

13. A compound of the formula:

$$CH_2(X)CH(Y)CH_2OH,$$

wherein either X and Y are different and are each iodine or bromine, or X and Y are different and are each fluorine or iodine.

14. 2-Bromo-3-chloro-1,4-butanediol.

15. A method as claimed in claim 1, wherein the end product is a compound as claimed in claim 13 or claim 14.

16. A method for the manufacture of an epoxide which comprises converting a vicinally, heterogeneously dihalogenated product which has been produced by a method as claimed in any one of claims 1 to 12 to an epoxide by reaction with slaked lime or enzymatically by means of a halohydrin epoxidase.

17. The use of a compound as claimed in claim 13 or claim 14 as a solvent, as a fumigant, e.g. a soil fumigant, as a pesticidal agent, as a refrigerant, as a flame retardant, or as a chemical intermediate.

**Patentansprüche**

1. Verfahren zur vicinalen, heterogenen Dihalogenierung von Alkenen und Alkinen durch gemeinsame Umsetzung einer Haloperoxidase, eines Oxidationsmittels, zweier verschiedener Halogenidionen, die beide zur Verwertung durch die Haloperoxidase befähigt sind und deren beider Gesamtkonzentration zusammen größer als 2000 mM ist, und eines zur Dihalogenierung durch die Haloperoxidase befähigten Alkens oder Alkins für einen zur Umwandlung des Alkens oder Alkins in ein vicinales heterogen dihalogeniertes Produkt ausreichenden Zeitraum.

2. Verfahren nach Anspruch 1, in dem die Haloperoxidase von dem Mikroorganismus *Caldariomyces fumago* (ATCC 16373), Algen, Milch, Thyroid, Leukozyten oder Meerrettich stammt.

3. Verfahren nach den Ansprüchen 1 oder 2, in dem das Oxidationsmittel Wasserstoffperoxid ist.

4. Verfahren nach Anspruch 3, in dem das Wasserstoffperoxid in einem Molverhältnis zum Alken oder Alkin von etwa 0,5:1 bis etwa 50:1 vorhanden ist.

5. Verfahren nach Anspruch 3 oder 4, in dem das Wasserstoffperoxid *in situ* erzeugt wird.

6. Verfahren nach einem der Ansprüche 1 bis 5, in dem die Halogenidionenquellen wasserlösliche Halogenidsalze sind, ausgewählt aus den Fluorid-, Chlorid-, Bromid- und Jodidsalzen von Natrium, Kalium und Lithium.

7. Verfahren nach einem der Ansprüche 1 bis 5, in dem die Halogenidionenquellen aus Meerwasser oder anderen natürlichen Kochsalzlösungen ausgewählt sind.

8. Verfahren nach einem der Ansprüche 1 bis 7, in dem die Umsetzung bei einem pH-Wert von etwa 2,8 bis etwa 8,0 durchgeführt wird.

9. Verfahren nach einem der Ansprüche 1 bis 8, in dem die zwei verschiedenen Halogenidionen aus einem Halogenidion, das in einem Maß vorhanden ist, das etwa der für den Einbau in das Produkt erforderlichen stöchiometrischen Menge entspricht, und einem zweiten Halogenidion, das in einem Maß vorhanden ist, der einem größeren Überschuß über die zum Einbau in das Produkt erforderliche Menge entspricht, bestehen.

10. Verfahren nach einem der Ansprüche 1 bis 9, in dem die Umsetzung in wäßriger Umgebung bei Umgebungsbedingungen der Temperatur und des Drucks durchgeführt wird.

11. Verfahren nach einem der Ansprüche 1 bis 10, in dem das Alken oder Alkin Äthylen, Allylchlorid, Allylalkohol, 2-Buten-1,4-diol oder 2-Butin-1,4-diol ist.

12. Verfahren nach Anspruch 1, in dem entweder die Haloperoxidase Lactoperoxidase und die zwei unterschiedlichen Halogenidionen Chlorid- und Bromidionen oder Chlorid- und Jodidionen sind, oder die Haloperoxidase Meerrettichperoxidase ist und die zwei unterschiedlichen Halogenidionen Bromid- und Jodidionen oder Chlorid- und Jodidionen oder Fluorid- und Jodidionen sind.

13. Eine Verbindung der Formel:

$$CH_2(X)CH(Y)CH_2OH$$

in der X und Y verschieden sind und jeweils Jod oder Brom bedeuten, oder X und Y verschieden sind und jeweils Fluor oder Jod bedeuten.

14. 2-Brom-3-chlor-1,4-butandiol.

15. Verfahren nach Anspruch 1, in dem das Endprodukt eine Verbindung nach Anspruch 13 oder Anspruch 14 ist.

16. Verfahren zur Herstellung eines Epoxids durch Umwandlung eines vicinal heterogen dihalogenierten Produktes, das nach einem Verfahren nach einem der Ansprüche 1 bis 12 hergestellt wurde, in ein Epoxid durch Umsetzung mit gelöschtem Kalk oder enzymatisch mit Hilfe einer Halohydrin-Epoxidase.

17. Verwendung einer Verbindung nach den Ansprüchen 13 oder 14 als Lösungsmittel, als Desinfektionsmittel, z.B. als Bodendesinfektionsmittel, als pestizider Wirkstoff, als Kühlmittel, als Flammverzögerer oder als chemisches Zwischenprodukt.

**Revendications**

1. Procédé pour la dihalogénation vicinale hétérogène d'alcènes et d'alcynes, qui comprend la réaction entre une haloperoxydase, un agent oxydant, deux ions halogénure différents qui sont tous deux capables d'être utilisés par l'haloperoxydase et desquels deux ensemble la concentration totale est supérieure à 2000 mM, et un alcène ou un alcyne susceptible de dihalogénation par l'haloperoxydase, pendant une durée suffisante pour convertir l'alcène ou l'alcyne en un produit dihalogéné vicinalement et hétérogènement.

2. Procédé suivant la revendication 1, dans lequel l'haloperoxydase est issue du micro-organisme *Caldariomyces fumago* (ATCC 16373), d'algues, de lait, de la thyroïde, de leucocytes ou du raifort.

3. Procédé suivant la revendication 1 ou 2, dans lequel l'agent oxydant est le peroxyde d'hydrogène.

4. Procédé suivant la revendication 3, dans lequel le peroxyde d'hydrogène est présent dans un rapport molaire à l'alcène ou à l'alcyne d'environ 0,5:1 à environ 50:1.

5. Procédé suivant la revendication 3 ou 4, dans lequel le peroxyde d'hydrogène est engendré *in situ*.

6. Procédé suivant l'une quelconque des revendications 1 à 5, dans lequel les sources d'ions halogénure sont des halogénures salins hydrosolubles choisis parmi les fluorures, chlorures, bromures et iodures de sodium, de potassium et de lithium.

7. Procédé suivant l'une quelconque des revendications 1 à 5, dans lequel les sources d'ions halogénure sont choisies parmi l'eau de mer et d'autres eaux salées naturelles.

8. Procédé suivant l'une quelconque des revendications 1 à 7, dans lequel la réaction est exécutée à un pH d'environ 2,8 à environ 8,0.

9. Procédé suivant l'une quelconque des revendications 1 à 8, dans lequel les deux ions halogénure différents consistent en un ion halogénure présent en une concentration qui fait à peu près la quantité stoechiométrique requise pour l'incorporation au produit et le second halogénure est présent en une concentration en excès majeur sur celle requise pour l'incorporation au produit.

10. Procédé suivant l'une quelconque des revendications 1 à 9, dans lequel la réaction est exécutée dans un milieu aqueux aux conditions ambiantes de température et de pression.

11. Procédé suivant l'une quelconque des revendications 1 à 10, dans lequel l'alcène ou l'alcyne est l'éthylène, le chlorure d'allyle, l'alcool allylique, le 2-butène-1,4-diol ou le 2-butyne-1,4-diol.

12. Procédé suivant la revendication 1, dans lequel soit l'haloperoxydase est la lactoperoxydase et les deux ions halogénure différents sont les ions chlorure et bromure ou les ions chlorure et iodure, soit l'haloperoxydase est la peroxydase de raifort et les deux ions halogénure différents sont les ions bromure et iodure, ou les ions chlorure et iodure ou bien les ions fluorure et iodure.

13. Composé de formule

$$CH_2(X)CH(Y)CH_2OH$$

où soit X et Y sont différents et représentent chacun l'iode ou le brome, soit X et Y sont différents et représentent chacun le fluor ou l'iode.

14. Le 2-bromo-3-chloro-1,4-butanediol.

15. Procédé suivant la revendication 1, dans lequel le produit final est un composé suivant la revendication 13 ou 14.

16. Procédé de préparation d'un époxyde, qui comprend la conversion d'un produit dihalogéné vicinalement et hétérogènement qui a été obtenu par un procédé suivant l'une quelconque des revendications 1 à 12 en un époxyde par réaction avec de la chaux éteinte ou par voie enzymatique au moyen d'une halohydrine époxydase.

17. Utilisation d'un composé suivant la revendication 13 ou 14 comme solvant, comme fumigant, par exemple comme fumigant du sol, comme pesticide, comme agent réfrigérant, comme agent ignifuge ou comme intermédiaire chimique.